# FASCICULE DE BREVET EUROPEEN

(11) **EP 3 142 682 B1**
(45) Date de publication et mention de la délivrance du brevet: **28.11.2018**
(21) Numéro de dépôt: 15721740.7
(22) Date de dépôt: 13.05.2015
(51) Int. Cl.: A61K 38/07, A61K 8/64, A61K 31/23, A61P 17/14, A61K 45/06

(54) **ASSOCIATION D'UN TÉTRAPEPTIDE ET D'UN GLYCÉRYL ESTER POUR LE TRAITEMENT DE L'ALOPÉCIE ANDROGÉNIQUE.**
ASSOZIATION EINES TETRAPEPTIDS UND EINES GLYCERYLESTERS ZUR BEHANDLUNG VON ANDROGENER ALOPEZIE
ASSOCIATION OF A TETRAPEPTIDE AND A GLYCERYL ESTER FOR TREATING ANDROGENIC ALOPECIA

(30) Priorité: 16.05.2014 FR 1454401
(43) Date de publication de la demande: 22.03.2017
(73) Titulaire: Pierre Fabre Dermo-Cosmétique, 92100 Boulogne-Billancourt (FR)
(72) Inventeur: POIGNY, Stéphane, F-31600 Saubens (FR); LEVEQUE, Marguerite, F-31400 Toulouse (FR); LEMERCIER, Laetitia, F-31200 Toulouse (FR)
(74) Mandataire: Regimbeau
(86) Numéro de dépôt international: PCT/EP2015/060644
(87) Numéro de publication internationale: WO 2015/173326

(56) Documents cités:
- WO-A1-92/21320
- WO-A1-2013/083825
- FR-A1- 2 857 597

## Description

La présente invention concerne une nouvelle association d'un conjugué peptidique contenant la séquence Lys-Gly-His-Lys et de glycéryl laurate ou un de ses dérivés, ainsi que l'utilisation de ladite association dans le domaine capillaire afin de lutter contre la miniaturisation du cheveu et traiter l'alopécie androgénique.

Le follicule pileux est un mini-organe ancré dans la peau jusqu'à l'hypoderme, et dont la fonction principale est la production d'une tige pilaire. Il est composé d'une gaine conjonctive externe (connective tissue sheath ou CTS) formée par des fibroblastes et des protéines de la matrice extracellulaire, d'une gaine épithéliale externe (outer root sheath ou ORS) et d'une gaine épithéliale interne (inner root sheath ou IRS) qui forment une paroi servant de guide à la tige pilaire lors de sa croissance. A l'extrémité proximale du follicule se trouve le bulbe pileux. Il est composé de la papille dermique, de mélanocytes et de cellules matricielles progénitrices des kératinocytes folliculaires de l'IRS et de la tige pilaire appelées cellules de la matrice. La papille dermique est constituée de fibroblastes spécialisés en contact avec le système vasculaire sanguin et joue un rôle essentiel dans la croissance pilaire. Le follicule pileux comprend également une réserve de cellules souches épithéliales et mélanocytaires située sous la glande sébacée et dans la gaine épithéliale externe: le bulge. Ces cellules sont caractérisées par un cycle cellulaire lent et une capacité à se différencier en plusieurs types cellulaires, dont les cellules de la matrice (K. Krause et K. Foitzik, Semin Cutan Med Surg, 2006; G. Cotsarelis, American Journal of Pathology, 1997).

Le cycle de croissance pilaire est représenté dans le temps par trois phases (anagène, catagène, télogène) dont la plus importante est la phase anagène ou phase de croissance de la tige pilaire.

L'alopécie androgénique est due à une accélération du cycle pilaire (raccourcissement de la durée du cycle pilaire), phénomène qui conduit dans un premier temps à l'apparition de cheveux miniaturisés dits « vellus » puis à un épuisement prématuré du renouvellement des cheveux. En effet, la durée de la phase anagène (phase de croissance) se raccourcie et passe de plusieurs années (2 à 5 ans) à quelques mois voire quelques semaines (Whiting et al, J Investig Dermatol Symp Proc, 1999). La conséquence est une chute précoce des cheveux.

Il est connu à ce jour que les mécanismes responsables de l'alopécie androgénique mettent entre autres en jeu la composante hormonale avec la surexpression du récepteur aux androgènes (= récepteur de la Testostérone et de la DHT) et une activité plus intense de l'enzyme 5 alpha-réductase. Cette dérégulation hormonale entraîne une production excessive de dihydrotestostérone, métabolite actif de la testostérone. Au niveau de la papille dermique, ce métabolite va stimuler la production d'inhibiteurs du cycle pilaire entraînant un raccourcissement de la phase anagène et nécessairement après plusieurs cycles, un épuisement de la capacité du follicule pileux à produire une tige pilaire.

Une propriété également valorisée dans le traitement général de la chute des cheveux vise à stimuler la production de VEGF, facteur de croissance angiogénique.

En effet, la papille dermique, structure contrôlant la croissance pilaire, se caractérise par la présence d'un réseau vasculaire très développé en phase anagène. Il a été démontré que les cellules de cette papille expriment durant ce stade et de façon intense, le facteur de croissance angiogénique VEGF (S.Lachgar M.Charveron in "Hair for the next millenium" - EdS. D, JJ Van Neste - VA Randall - H. Baden - H. Ogawa et R. Oliver - Elsevier - Amsterdam - 1996 - p. 407-412). Ce facteur de croissance vasculaire endothélial permet le maintien de la vascularisation nécessaire à la croissance du néo-cheveu et son développement pendant toute la phase anagène.

Les divers régulateurs du cycle pilaire voient leur expression modulée par différentes voies de signalisation cellulaires, que ce soit pour activer la croissance et la différenciation cellulaire au cours de la phase anagène ou pour la mise en place d'une apoptose massive au passage en phase catagène. Ces voies sont elles-mêmes régulées par des inducteurs et des inhibiteurs dont les niveaux varient en fonction des phases du cycle, permettant ainsi des transitions de phases en phases parfaitement maîtrisées.

Récemment, certains travaux suggèrent un rôle majeur de la voie Wnt/bêta-caténine pour assurer un cycle pilaire normal (Lien et al, Nature Cell Biology, 2014). Cette voie de signalisation serait en effet fortement impliquée dans des phénomènes de haute prolifération cellulaire, notamment au cours de l'embryogénèse et de la phase de croissance du cycle pilaire. Une déficience de cette voie entraînerait une perturbation du développement du follicule pileux en phase anagène.

L'invention vise à proposer de nouvelles compositions cosmétiques ou dermatologiques qui permettraient de normaliser la signalisation Wnt/bêta-caténine (voie Wnt canonique), laquelle joue un rôle majeur dans la régénération des cellules du follicule pileux, et seraient utilisées dans le traitement de l'alopécie androgénique.

La demanderesse a procédé à une étude réalisée sur des prélèvements de follicules pileux arrachés auprès de sujets alopéciques (alopécie androgénique). Cette étude établit l'existence d'une relation étroite entre la perte de densité des cheveux dans l'AAG et la perturbation de la voie de signalisation Wnt/bêta-caténine. Cette étude démontre que l'amplification du gène codant pour un inhibiteur de la voie Wnt/bêta-caténine, conduit à une élévation de la protéine correspondante dans les prélèvements folliculaires des sujets alopéciques ce qui apparait aller dans le sens d'une implication de la dérégulation de la voie Wnt/bêta-caténine dans l'apparition de l'alopécie androgénique.

La demanderesse a mis en évidence l'existence d'une synergie entre un peptide contenant la séquence Lys-Gly-His-Lys et du glycéryl laurate ou un de ses dérivés pour inhiber le niveau d'expression du gène SFRP1 codant pour un inhibiteur de la voie Wnt/bêta-caténine et pour stimuler le niveau d'expression du gène FZD1 codant pour un récepteur des ligands Wnt responsable de l'activation de la voie Wnt/bêta-caténine dans les cellules de la papille dermique et les cellules de la matrice (Reddy et al, J Invest Dermatol., 2004).

De plus il a également été démontré une synergie d'action de cette association d'actifs pour activer le niveau d'expression génique de kératines spécifiques (dont KRT17, KRT25, KRT27 et KRT81) dont l'expression est régulée par la voie Wnt/bêta-caténine, ces kératines étant des marqueurs de la différenciation des cellules souches épithéliales du bulbe en kératinocytes folliculaires.

Plus spécifiquement, cette association d'actifs va normaliser le renouvellement du cycle pilaire en rétablissant le fonctionnement de la voie Wnt/bêta-caténine.

Ainsi cette association d'actifs va permettre de lutter contre le raccourcissement des cycles pilaires (agir comme véritable « prolongateur » de croissance pilaire) et la miniaturisation du cheveu.

Ainsi, la présente invention a pour objet une association comprenant un peptide contenant la séquence A-Lys-Gly-His-Lys-NH2 dans laquelle A représente le radical correspondant à un acide gras saturé ou insaturé en C1-C18 ; et du glycéryl laurate ou un de ses dérivés.

Dans le peptide contenant la séquence A-Lys-Gly-His-Lys-NH2, « A » représente préférentiellement le radical acétyle, palmityle ou stéaryle.

Un tel peptide est par exemple décrit dans le document EP 1 663 285.

Dans un mode de réalisation particulier, le conjugué peptidique est l'acétyl tétrapeptide-3, lequel a pour séquence : A-Lys-Gly-His-Lys-NH2 dans laquelle A est un groupe acétyle ; le numéro CAS correspondant étant le 827306 - 88 - 7.

Le tétrapeptide va stimuler la microcirculation du cuir chevelu et faciliter l'apport d'éléments nécessaires au métabolisme cellulaire.

La synergie a été observée avec le glycéryl laurate en tant qu'inhibiteur de 5 alpha réductase, mais cet effet est élargi aux dérivés / précurseurs du glycéryl laurate de formule I suivante 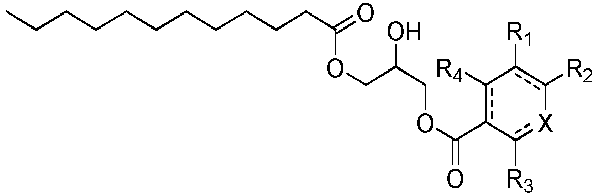 dans laquelle
- si X = NH, alors chacun des R₁, R₂, R₃, R₄ représente un atome d'hydrogène ;
- si X = N, alors le noyau est aromatique et R₁, R₂, R₃ et R₄ représentent des atomes d'hydrogène, ou l'un d'entre eux un groupement méthyle ; et lorsque R₂=R₃=R₄=H, R₁ peut aussi représenter un atome d'halogène ou un radical aryle, hétéroaryle, alcényle, acétylényle.

Ces dérivés sont décrits par exemple dans la demande WO2013/083825.

Selon un mode particulier de l'invention, le rapport massique entre le glycéryl laurate et le peptide est compris entre 0,5 et 300, de préférence entre 1 et 200 et plus préférentiellement de l'ordre de 100.

La présente invention porte, en outre, sur une association comprenant un peptide contenant la séquence A-Lys-Gly-His-Lys-NH2 dans laquelle A représente le radical correspondant à un acide gras saturé ou insaturé en C1-C18 ; et du glycéryl laurate ou un de ses dérivés pour son utilisation topique destinée à traiter l'alopécie androgénique ; ainsi que sur l'utilisation de l'association pour favoriser la croissance capillaire.

La présente invention concerne également une composition cosmétique ou dermatologique comprenant, en tant que principe actif antichute cheveu, une association comprenant un peptide contenant la séquence A-Lys-Gly-His-Lys dans laquelle A représente le radical correspondant à un acide gras saturé ou insaturé en C1-C18 et du glycéryl laurate ou un de ses dérivés et comprenant en outre au moins un excipient cosmétiquement ou dermatologiquement acceptable.

De préférence, les excipients acceptables sont adaptés à une administration topique.

Les excipients acceptables permettent en particulier d'assurer une bonne stabilité, une texture et un toucher agréables. Il peut aussi s'agir par exemple d'agents de formulations ou d'additifs d'usage connu et classique en cosmétique : on peut citer des tensioactifs, colorants, conservateurs, parfums, agents filmogènes, etc.

Les compositions selon l'invention peuvent se présenter sous les formes qui sont habituellement connues pour une administration topique sur les cheveux et le cuir chevelu, c'est-à-dire notamment un shampoing, un après shampoing, une crème capillaire, une lotion capillaire ou un spray sans rinçage.

Dans un mode de réalisation préféré, la composition sera sous forme d'un spray sans rinçage.

L'objet de la présente invention vise l'utilisation cosmétique de l'association selon la présente invention ou de cette composition cosmétique selon l'invention pour favoriser la croissance capillaire. L'utilisation cosmétique de l'association selon la présente invention ou la composition cosmétique selon l'invention est plus particulièrement destinée à réguler le cycle pilaire et favoriser la régénération folliculaire.

La présente invention concerne également un procédé de soin cosmétique des cheveux destiné à améliorer l'esthétique des cheveux en favorisant la pousse capillaire caractérisé en ce qu'il consiste à appliquer sur les cheveux et le cuir chevelu une quantité efficace d'une association selon l'invention ou d'une composition cosmétique selon l'invention, à laisser celle-ci au contact des cheveux, et éventuellement à rincer les cheveux.

Un autre objet de la présente invention concerne une composition dermatologique pour son utilisation dans le traitement de l'alopécie androgénique comprenant en tant que principe actif l'association selon l'invention.

Dans un mode de réalisation préférée de l'invention, la composition est destinée à une application topique.

Dans un mode de réalisation particulier, les compositions dermatologiques et cosmétiques selon l'invention comprennent au moins un autre principe actif.

Cet autre principe actif pourra notamment être sélectionné dans le groupe comprenant d'autres inhibiteurs de 5 alpha réductase.

Selon un mode de réalisation particulier, les compositions selon l'invention comprennent en outre un dérivé nicotinique dont la vitamine PP (Nicotinamide) ou un ester choisis parmi les nicotinates de méthyle, d'éthyle, de 2-éthylhexyle, miristyle et de benzyle ainsi que le nicotinate d'alpha-tocophérol ou le nicotinate de delta-tocophérol.

De préférence, cet actif antichute complémentaire est le nicotinate d'alpha-tocophérol. Il agit en tant qu'enhancer de pénétration pour les autres actifs, il stimule le métabolisme cellulaire et favorise la microcirculation cutanée (stimulation de la production de VEGF).

La composition selon l'invention comprend alors une association comprenant un peptide contenant la séquence A-Lys-Gly-His-Lys dans laquelle A représente le radical correspondant à un acide gras saturé ou insaturé en C1-C18; du glycéryl laurate ou un de ses dérivés et de nicotinate d'alpha-tocophérol.

Selon un autre mode de réalisation de l'invention, la composition comprendra en outre le diguanosine tétraphosphate ou GP4G.

Il s'agit d'un principe actif de biotechnologie marine extrait de zooplancton : Artemia Salina, et répondant à la structure chimique suivante :

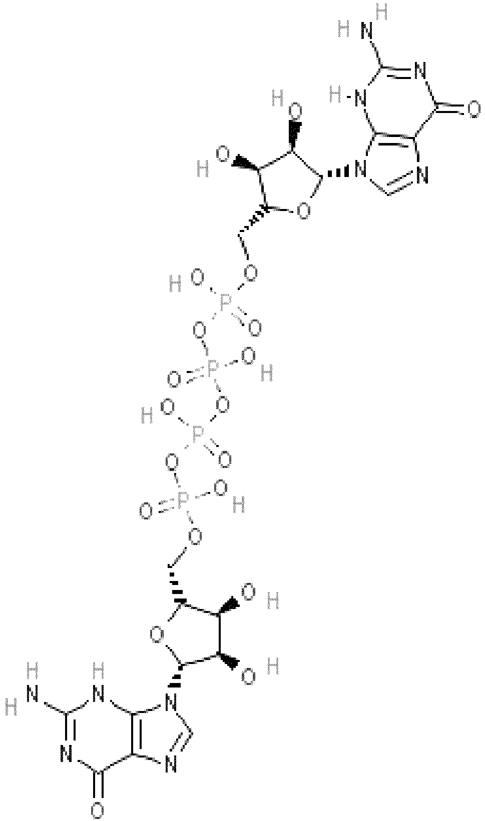

La composition selon l'invention comprend alors une association comprenant un peptide contenant la séquence A-Lys-Gly-His-Lys ; du glycéryl laurate ou un de ses dérivés et du GP4G.

Dans un mode de réalisation préféré, l'association comprendra en outre du nicotinate de tocophérol et du GP4G ; ces 2 actifs complémentaires agissant en tant que complexe vasculotrope (voir EP1336402).

Dans un mode de réalisation préféré, le complexe nicotinate de tocophérol et GP4G est présent dans les quantités suivantes
- entre 0,05 % et 5 % en poids de nicotinate de tocophérol, de préférence de 0,1 à 2,5%
- entre 0,01 % et 5 % en poids de GP4G ; de préférence de 0,05 à 2,5% par rapport au poids total de la composition.

Dans un mode de réalisation préféré, la composition finale sera transparente et de préférence sous forme de lotion transparente.

Par "composition transparente", on entend une composition homogène (une seule phase) et limpide laissant passer la lumière, par exemple de manière à permettre la lecture, à travers un flacon, du verso d'une étiquette appliquée sur ledit flacon de verre ou de matière plastique également transparente contenant ladite composition.

A cette fin, il a été mis en évidence que le solvant à utiliser en complément de l'éthanol (10 à 40%) intervenant en tant qu'agent facilitant la pénétration des produits antichute de cheveux, est l'alcool isopropylique en quantité comprise entre 1% et 5% ; et de préférence à 2.5% en poids par rapport au poids total de la composition. Il a été remarqué que l'alcool isopropylique (2,5%) joue, à matière active 4 fois plus faible que l'hexylène glycol (10%) classiquement utilisé pour améliorer la pénétration dans le follicule pileux, le rôle de solubilisant des actifs cireux lipophiles que sont le nicotinate de tocophéryl et le glycéryl laurate ; action solvante indispensable pour la qualité visuelle du produit mais aussi pour augmenter la biodisponibilité de ces deux actifs agissant respectivement sur l'augmentation du flux sanguin et la régulation de l'activité de la 5 alpha réductase.

La texture légère de ladite composition permet en outre une pénétration optimale sans graisser les cheveux. Dès les premières applications, la chevelure retrouve force et vitalité.

La composition selon l'invention permet d'enrayer la chute du cheveu, d'en prolonger son cycle. Le capital cheveu est préservé, en quantité et en qualité.

Les exemples qui suivent sont destinés à illustrer certains modes de réalisation particuliers de l'invention et représentent en particulier certaines compositions utilisables pour la mise en oeuvre de l'invention. Les excipients mentionnés dans les exemples de composition ne sont donnés qu'à titre illustratif; et il est à la portée de l'homme du métier d'en substituer d'autres.

### MATERIEL ET METHODES

Les bulbes de follicules pileux issus de prélèvements de chirurgie plastique (lifts faciaux) de deux donneurs ont été isolés par microdissection, mis en culture et traités pendant 6h avec les actifs (acétyl tétrapeptide-3, Glycéryl laurate et leur association). Les ARN ont été extraits, dosés, rétro-transcrits et l'expression d'un panel de gènes exprimés dans le follicule pilo-sébacé a été analysée par PCR quantitative en temps réel.

L'analyse des résultats générés dans cette étude a conduit, pour chacune des conditions travaillées et pour chacun des gènes d'intérêt étudiés, au calcul d'une quantité relative (QR). Celle-ci correspond au niveau d'expression d'un gène comparé à son niveau d'expression basal (niveau d'expression obtenu pour la condition Témoin dont la QR est ramenée à 1). Ainsi, la modulation du niveau d'expression d'un gène est considérée significative lorsque :
- la QR est supérieure ou égale à 2 : gène deux fois plus exprimé par rapport à la condition Témoin
- la QR est inférieure ou égale à 0,5 : gène deux fois moins exprimé par rapport à la condition Témoin.

Les QR ne pouvant pas s'additionner, un effet est considéré comme synergique lorsque la QR obtenue avec l'association d'actifs est significativement supérieure ou inférieure aux QR obtenues pour chacun des actifs testés seuls.

L'ensemble des résultats présentés ci-dessous correspond aux résultats moyennés obtenus pour les deux donneurs de l'étude.

### RESULTATS

Parmi l'ensemble des gènes étudiés, 11 gènes d'intérêt sont favorablement régulés par l'association de l'Acétyl tétrapeptide-3 (ATP-3) et du Glycéryl laurate (GL), selon un effet synergique.

L'association de l'Acétyl tétrapeptide-3 et du Glycéryl laurate stimule le niveau d'expression des gènes COL3A1 et COL7A1 (impliqués dans l'ancrage du follicule pileux), ainsi que l'expression du gène IGF1R (impliqué dans le développement du follicule pileux en phase anagène). Elle semble également induire l'expression du gène SHH et inhiber l'expression du gène BMP4, tous deux impliqués dans la signalisation Sonic Hedgehog qui contribue à l'auto-renouvellement, la prolifération et la différenciation des cellules souches du follicule pileux.

Modulation selon un effet synergique du niveau d'expression de gènes impliqués dans la voie de signalisation Wnt/bêta-caténine par l'association de l'Acétyl tétrapeptide-3 et du Glycéryl laurate.

Cette voie de signalisation est décrite pour être fortement impliquée dans la régulation du cycle pilaire (transition télogène → anagène) et pour jouer un rôle essentiel dans le maintien des follicules pileux en phase anagène et la production d'une nouvelle tige pilaire. En effet, elle stimule la prolifération et la différenciation des cellules souches épithéliales du bulge et des cellules progénitrices en kératinocytes folliculaires. La voie Wnt/bêta-caténine est décrite pour être inhibée par l'action des androgènes d'où l'intérêt que représente la restauration de ce signal dans le traitement de l'alopécie androgénique.

Le récepteur « frizzled family receptor 1 » (codé par le gène FZD1) est un récepteur transmembranaire des voies Wnt canonique (Wnt/bêta-caténine) et non canonique. Au niveau du follicule pileux, il est exprimé par les cellules de l'ORS, les cellules de la matrice et les cellules de la papille dermique. Son niveau d'expression, faible en phase télogène, est augmenté en début de phase anagène (anagen onset). Au niveau de l'ORS, les ligands Wnt majoritairement exprimés sont les ligands Wnt5a et Wnt11, décrits pour activer la voie Wnt non canonique, ce qui pourrait permettre de réguler la migration des cellules souches progénitrices du bulge vers la matrice. Au niveau de la matrice et de la papille dermique, les ligands Wnt majoritairement exprimés sont les ligands Wnt10a et Wnt10b, décrits pour activer la voie Wnt/bêta-caténine.

Après 6 heures de traitement, l'association de l'Acétyl tétrapeptide-3 (ATP-3) et du Glycéryl laurate (GL) stimule selon un effet synergique le niveau d'expression du gène FZD1.

Le suivi du niveau d'expression du gène FZD1 dans les follicules pileux humains est donné dans le tableau I ci-dessous :

**TABLEAU I**

| | 6h | | | |
|---|---|---|---|---|
| | Témoin | ATP-3 | GL | ATP-3 + GL |
| | - | 20µM | 10µM | 20µM + 10 µM |
| QR | 1.0 | 1.2 | 1.3 | 4.4 |

La protéine « secreted frizzled-related protein 1 » (codée par le gène SFRP1) est un inhibiteur de la voie Wnt/bêta-caténine. Elle possède un domaine homologue au site de liaison Wnt sur les récepteurs frizzled, elle est donc capable de se lier aux protéines Wnt et d'empêcher leur fixation au niveau des récepteurs frizzled, mais également capable de former un complexe inhibiteur avec les récepteurs frizzled et d'empêcher ainsi l'activation de la signalisation Wnt.

L'article de Karnik et al (Microarray analysis of androgenetic and senescent alopecia : Comparison of gene expression shows two distinct profiles, Journal of Dermatological Science 72 (2013) 183-183) décrit différents marqueurs spécifiques de l'alopécie androgénique (AAG), parmi lesquels le gène codant l'inhibiteur SFRP1 qui est retrouvé surexprimé d'un facteur 3.73 chez les patients AAG comparé au groupe contrôle.

Après 6 heures de traitement, l'association de l'Acétyl tétrapeptide-3 (ATP-3) et du Glycéryl laurate (GL) inhibe selon un effet synergique le niveau d'expression du gène SFRP1.

Le suivi du niveau d'expression du gène SFRP1 dans les follicules pileux humains est donné dans le tableau II ci-dessous :

**TABLEAU II**

| | 6h | | | |
|---|---|---|---|---|
| | Témoin | ATP-3 | GL | ATP-3 + GL |
| | - | 20µM | 10µM | 20µM + 10 µM |
| QR | 1.0 | 1.3 | 0.9 | 0.4 |

Au cours de la transition télogène-anagène et en début de phase anagène, les cellules souches épithéliales du bulge se différencient en kératinocytes folliculaires afin de générer les couches concentriques formant l'ORS, l'IRS et la tige pilaire nécessaires à la régénération du follicule pileux. Chaque couche constituant le follicule est caractérisée par un type de kératinocyte produisant un panel de protéines, majoritairement des kératines, qui lui est propre. Les kératines sont donc des marqueurs de cette différenciation et leur expression est, pour la plupart, régulée par la voie de signalisation Wnt/bêta-caténine.

La kératine 17, codée par le gène KRT17, est exprimée par les kératinocytes folliculaires de l'ORS. Les kératines 25 et 27, codées respectivement par les gènes KRT25 et KRT27, sont exprimées par les kératinocytes folliculaires de l'IRS. La kératine 81, codée par le gène KRT81, est exprimée par les kératinocytes folliculaires du cortex.

L'article de Karnik et al (Microarray analysis of androgenetic and senescent alopecia : Comparison of gene expression shows two distinct profiles, Journal of Dermatological Science 72 (2013) 183-183) a également mis en évidence la kératine 27 comme étant un marqueur spécifique de l'AAG, le gène KRT27 étant retrouvé sous-exprimé d'un facteur 4.92 chez les patients AAG comparé au groupe contrôle.

Après 6 heures de traitement, l'association de l'Acétyl tétrapeptide-3 (ATP-3) et du Glycéryl laurate (GL) stimule selon un effet synergique le niveau d'expression génique des kératines 17, 25, 27 et 81.

Le suivi du niveau d'expression du gène KRT17 dans les follicules pileux humains est donné dans le tableau III ci-dessous :

**TABLEAU III**

| | 6h | | | |
|---|---|---|---|---|
| | Témoin | ATP-3 | GL | ATP-3 + GL |
| | - | 20µM | 10µM | 20µM + 10 µM |
| QR | 1.0 | 0.8 | 1.1 | 2.3 |

Le suivi du niveau d'expression du gène KRT 25 dans les follicules pileux humains est donné dans le tableau IV ci-dessous :

**TABLEAU IV**

| | 6h | | | |
|---|---|---|---|---|
| | Témoin | ATP-3 | GL | ATP-3 + GL |
| | - | 20µM | 10µM | 20µM + 10 µM |
| QR | 1.0 | 1.7 | 1.3 | 6.8 |

Le suivi du niveau d'expression du gène KRT 27 dans les follicules pileux humains est donné dans le tableau V ci-dessous :

**TABLEAU V**

| | 6h | | | |
|---|---|---|---|---|
| | Témoin | ATP-3 | GL | ATP-3 + GL |
| | - | 20µM | 10µM | 20µM + 10 µM |
| QR | 1.0 | 1.4 | 1.3 | 3.2 |

Le suivi du niveau d'expression du gène KRT 81 dans les follicules pileux humains est donné dans le tableau VI ci-dessous :

**TABLEAU VI**

| | 6h | | | |
|---|---|---|---|---|
| | Témoin | ATP-3 | GL | ATP-3 + GL |
| | - | 20µM | 10µM | 20µM + 10 µM |
| QR | 1.0 | 1.5 | 1.8 | 4.0 |

On mentionnera ci-après, à simple titre d'illustration de l'objet de l'invention deux exemples de lotions transparentes :

### Exemple 1 : lotion transparente

ACETYL TETRAPEPTIDE-3 PP de 0,001 à 0,01 %
LAURATE GLYCERYL de 0,05 à 0,3%
GP4G de 0,01 à 5%
NICOTINATE TOCOPHERYL 0,05 à 5%
DEXPANTHENOL de 0,3 à 1%
ALCOOL ISOPROPYLIQUE de 1 à 5%
PPG-26-BUT.-26/PEG-40 de 2 à 10%
ETHYLIQUE ALCOOL de 10 à 40%
PARFUM qsp
EAU qsp

### Exemple 2 : lotion transparente

ACETYL TETRAPEPTIDE-3 PP de 0,001 à 0,01 %
LAURATE GLYCERYL de 0,05 à 0,3%
GP4G de 0,01 à 5%
NICOTINATE TOCOPHERYL 0,05 à 5%
DEXPANTHENOL de 0,3 à 1%
BETAINE HYDRATEE de 0,5 à 3%
ALCOOL ISOPROPYLIQUE de 1 à 5%
PPG-26-BUT.-26/PEG-40 de 2 à 10%
ETHYLIQUE ALCOOL de 10 à 40%
PARFUM qsp
EAU qsp

## Revendications

1. Association comprenant un peptide contenant la séquence A-Lys-Gly-His-Lys-NH₂, dans laquelle A représente le radical correspondant à un acide gras saturé ou insaturé en C₁ à C₁₈, et du glycéryl laurate ou un de ses dérivés, **caractérisé en ce que** lesdits dérivés correspondent à la formule I suivante : 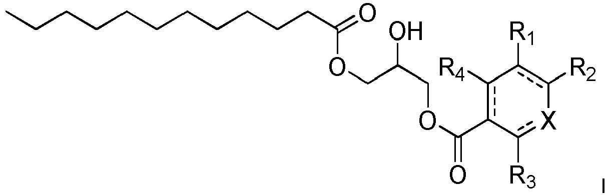 dans laquelle
- si X = NH, alors chacun des R₁, R₂, R₃, R₄ représente un atome d'hydrogène ;
- si X = N, alors le noyau est aromatique et R₁, R₂, R₃ et R₄ représentent des atomes d'hydrogène, ou l'un d'entre eux un groupement méthyle ; et lorsque R₂=R₃=R₄=H, R₁ peut aussi représenter un atome d'halogène ou un radical aryle, hétéroaryle, alcényle, acétylényle.

2. Association selon la revendication 1, **caractérisée en ce que** A représente le radical acétyle, palmityle ou stéaryle.

3. Association selon la revendication 1 ou 2, **caractérisée en ce que** le rapport massique entre le glycéryl laurate et le peptide est compris entre 0,5 et 300, de préférence entre 1 et 200 et plus préférentiellement de l'ordre de 100.

4. Association selon l'une des revendications 1 à 3, pour son utilisation topique destinée à traiter l'alopécie androgénique.

5. Composition dermatologique ou cosmétique comprenant à titre de principe actif une association selon l'une des revendications 1 à 4, avec au moins un excipient dermatologiquement ou cosmétiquement acceptable.

6. Composition selon la revendication 5, **caractérisée en ce que** le peptide ou conjugué peptidique est présent à une teneur de 0,001% à 0,01% en poids de la composition totale.

7. Composition selon l'une des revendications 5 et 6, en ce que le glycéryl laurate ou dérivé de ce dernier est présent à une teneur de 0,05% à 0,3% en poids de la composition totale.

8. Composition selon l'une des revendications 5 à 7, **caractérisée en ce qu'**un des excipients est l'alcool isopropylique comme solvant présent en une quantité comprise entre 1% et 5%, de préférence à 2,5%, en poids par rapport au poids total de la composition.

9. Composition selon l'une des revendications 5 à 8, **caractérisée en ce qu'**elle comprend en outre un autre principe actif.

10. Composition selon la revendication 9, **caractérisée en ce que** l'autre principe actif est choisi parmi d'autres inhibiteurs de 5 alpha réductase et leurs mélanges.

11. Composition selon la revendication 9, **caractérisée en ce que** l'autre principe actif est du diguanosine tétraphosphate.

12. Composition selon la revendication 9, **caractérisée en ce que** l'autre principe actif est du nicotinate de tocophérol.

13. Composition dermatologique selon l'une des revendications 5 à 12, pour son utilisation dans le traitement de l'alopécie.

## Patentansprüche

1. Assoziation, die ein Peptid, das die Sequenz A-Lys-Gly-His-Lys-NH₂ enthält, in der A das Radikal darstellt, das einer gesättigten oder ungesättigten C₁-bis C₁₈-Fettsäure entspricht, und Glyceryllaurat oder eines seiner Derivate umfasst, **dadurch gekennzeichnet, dass** die Derivate der folgenden Formel I entsprechen: 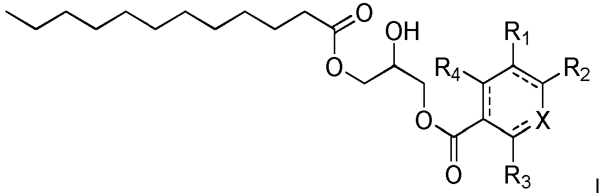 wobei:
- wenn X = NH, jedes von R₁, R₂, R₃, R₄ ein Wasserstoffatom darstellt;
- wenn X = N, der Kern aromatisch ist und R₁, R₂, R₃ und R₄ Wasserstoffatome oder eines davon eine Methylgruppe darstellen; und wenn R₂=R₃=R₄=H, R₁ auch ein Halogenatom oder ein Aryl-, Heteroaryl-, Alkenyl-, Acetylenylradikal darstellen kann.

2. Assoziation nach Anspruch 1, **dadurch gekennzeichnet, dass** A das Acetyl-, Palmityl- oder Stearylradikal darstellt.

3. Assoziation nach Anspruch 1 oder 2, **dadurch gekennzeichnet, dass** das Massenverhältnis zwischen dem Glyceryllaurat und dem Peptid zwischen 0,5 und 300, vorzugsweise zwischen 1 und 200 und mehr zu bevorzugen in der Größenordnung von 100 enthalten ist.

4. Assoziation nach einem der Ansprüche 1 bis 3 für ihre topische Verwendung, die zur Behandlung von androgenetischer Alopezie bestimmt ist.

5. Dermatologische oder kosmetische Zusammensetzung, die als Wirkstoff eine Assoziation nach einem der Ansprüche 1 bis 4 mit mindestens einem dermatologisch oder kosmetisch annehmbaren Trägerstoff umfasst.

6. Zusammensetzung nach Anspruch 5, **dadurch gekennzeichnet, dass** das Peptid oder Peptidkonjugat mit einem Gehalt von 0,001 bis 0,01 Gew.-% der Gesamtzusammensetzung vorhanden ist.

7. Zusammensetzung nach einem der Ansprüche 5 und 6, **dadurch gekennzeichnet, dass** das Glyceryllaurat oder dessen Derivat mit einem Gehalt von 0,05 bis 0,3 Gew.-% der Gesamtzusammensetzung vorhanden ist.

8. Zusammensetzung nach einem der Ansprüche 5 bis 7, **dadurch gekennzeichnet, dass** einer der Trägerstoffe Isopropylalkohol als Lösungsmittel ist, das in einer Menge zwischen 1 und 5 Gew.-%, vorzugsweise 2,5 Gew.-%, im Verhältnis zum Gesamtgewicht der Zusammensetzung vorhanden ist.

9. Zusammensetzung nach einem der Ansprüche 5 bis 8, **dadurch gekennzeichnet, dass** sie ferner einen anderen Wirkstoff umfasst.

10. Zusammensetzung nach Anspruch 9, **dadurch gekennzeichnet, dass** der andere Wirkstoff unter anderen 5-Alphareduktasehemmern und ihren Mischungen ausgewählt ist.

11. Zusammensetzung nach Anspruch 9, **dadurch gekennzeichnet, dass** der andere Wirkstoff Diguanosintetraphosphat ist.

12. Zusammensetzung nach Anspruch 9, **dadurch gekennzeichnet, dass** der andere Wirkstoff Tocopherol-Nicotinat ist.

13. Zusammensetzung nach einem der Ansprüche 5 bis 12 für ihre Verwendung bei der Behandlung von Alopezie.

## Claims

1. Association comprising a peptide containing the sequence A-Lys-Gly-His-Lys-NH₂, wherein A represents the radical corresponding to a C₁-C₁₈ saturated or unsaturated fatty acid, and glyceryl laurate or a derivative thereof, **characterized in that** said derivatives correspond to the following formula I: 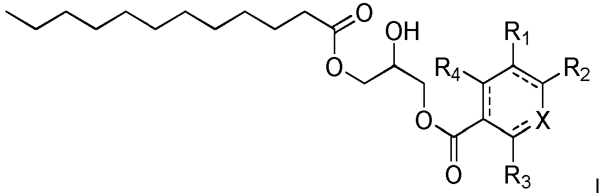 wherein
- if X = NH, then each of R₁, R₂, R₃, R₄ represents a hydrogen atom;
- if X = N, then the ring is aromatic and R₁, R₂, R₃ and R₄ represent hydrogen atoms, or one of which a methyl group; and when R₂=R₃=R₄=H, R₁ may also represent a halogen atom or an aryl, heteroaryl, alkenyl, acetylenyl radical.

2. Association according to claim 1, **characterized in that** A represents the acetyl, palmityl or stearyl radical.

3. Association according to claim 1 or 2, **characterized in that** the mass ratio between the glyceryl laurate and the peptide is between 0.5 and 300, preferably between 1 and 200 and more preferentially about 100.

4. Association according to one of claims 1 to 3, for topical use to treat androgenic alopecia.

5. Dermatological or cosmetic composition comprising as active agent an association according to one of claims 1 to 4, with at least one dermatologically or cosmetically acceptable excipient.

6. Composition according to claim 5, **characterized in that** the peptide or peptide conjugate is present in an amount of 0.001% to 0.01% by weight of the total composition.

7. Composition according to one of claims 5 and 6, **characterized in that** the glyceryl laurate or derivative thereof is present in an amount of 0.05% to 0.3% by weight of the total composition.

8. Composition according to one of claims 5 to 7, **characterized in that** one of the excipients is isopropyl alcohol as solvent present in an amount ranging between 1% and 5%, preferably 2.5%, by weight relative to the total weight of the composition.

9. Composition according to one of claims 5 to 8, **characterized in that** it further comprises one other active agent.

10. Composition according to claim 9, **characterized in that** the other active agent is selected from other 5-alpha-reductase inhibitors and mixtures thereof.

11. Composition according to claim 9, **characterized in that** the other active agent is diguanosine tetraphosphate.

12. Composition according to claim 9, **characterized in that** the other active agent is tocopherol nicotinate.

13. Dermatological composition according to one of claims 5 to 12, for use in the treatment of alopecia.
